(19) Europäisches Patentamt
European Patent Office
Office européen des brevets

(11) Publication number : **0 455 399 A2**

(12) # EUROPEAN PATENT APPLICATION

(21) Application number : **91303633.1**

(22) Date of filing : **23.04.91**

(51) Int. Cl.$^5$ : **C25B 3/08,** C07C 21/18

(30) Priority : **24.04.90 US 513774**

(43) Date of publication of application :
**06.11.91 Bulletin 91/45**

(84) Designated Contracting States :
**BE DE FR GB IT NL**

(71) Applicant : **MINNESOTA MINING AND MANUFACTURING COMPANY
3M Center, P.O. Box 33427
St. Paul, Minnesota 55133-3427 (US)**

(72) Inventor : **Childs, William Ves, c/o Minnesota Mining and
Manufact. Co., 2501 Hudson Road, P.O. Box 33427
St. Paul, Minnesota 55133-3427 (US)**
Inventor : **Herzberg, Thomas C., c/o Minnesota Mining and
Manufact. Co., 2501 Hudson Road, P.O. Box 33427
St. Paul, Minnesota 55133-3427 (US)**
Inventor : **Klink,Frank W.
c/o Minnesota Min.and/man.Co.,2501 Hudson Road,P.O
Box 33427/St Paul,Minnesota 55133-3427 (US)**
Inventor : **Kolpin,Charles F.
c/o Minnesota min.and man.Co.,2501 Hudson Road,P.O
.Box 33427/St.Paul,Minnesota 55133-3427 (US)**

(74) Representative : **Baillie, Iain Cameron et al
c/o Ladas & Parry Isartorplatz 5
W-8000 München 2 (DE)**

(54) **Process for making organic fluorine compounds.**

(57) 1,1,2,2-tetrafluorocyclobutane is fluorinated by Simons electrochemical fluorination process to produce octafluorocyclobutane, which can be pyrolized to produce hexafluoropropylene ; some of the tetrafluorocyclobutane can also be pyrolized to produce vinylidene fluoride.

EP 0 455 399 A2

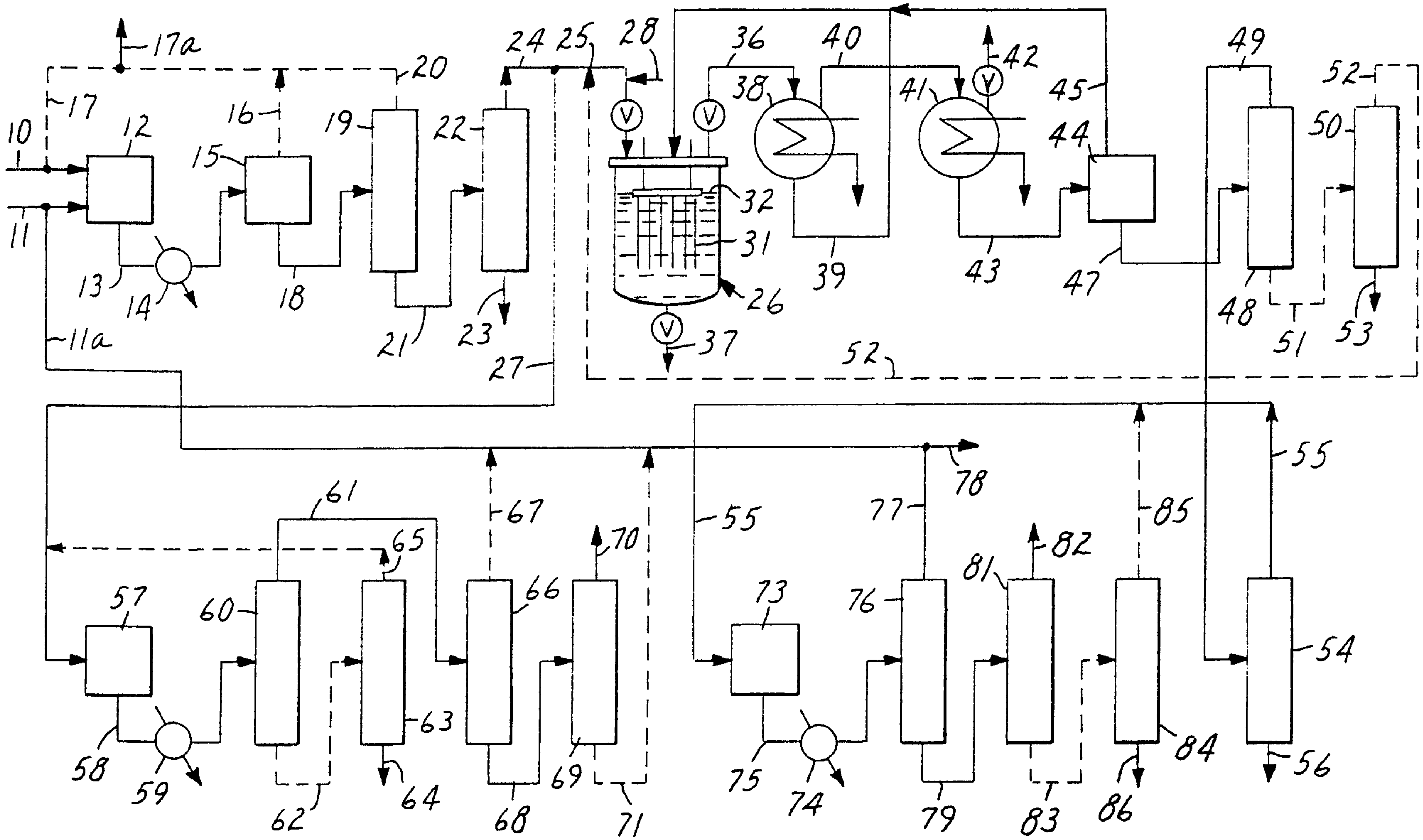
10
11
11a
12
13
14
15
16
17
17a
18
19
20
21
22
23
24
25
26
27
28
31
32
36
37
38
39
40
41
42
43
44
45
47
48
49
50
51
52
53
54
55
56
57
58
59
60
61
62
63
64
65
66
67
68
69
70
71
73
74
75
76
77
78
79
81
82
83
84
85
86

This invention relates to processes for making fluoroolefins, such as hexafluoropropylene, vinylidene fluoride, and tetrafluoroethylene. In another aspect, it relates to processes for making fluorocyclobutanes, such as 1,1,2,2-tetrafluorocyclobutane and octafluorocyclobutane, and their pyrolysis to make said fluoroolefins. Tetrafluoroethylene, hexafluoropropylene, and vinylidene fluoride are fluoroolefins useful as free-radical polymerizable monomers in the manufacture of such polymers as fluoroelastomers. Fluoroelastomers are an important class of rubber polymers from which various shaped articles such as 0-rings, gaskets, and protective coatings can be made with excellent thermal, chemical, and oxidative stabilities which enables them to withstand high temperatures of and exposure to harsh environments that quickly degrade other organic polymers.

Two precursors that are used to commercially manufacture fluoroolefins for such fluoroelastomers are hydrochlorofluorocarbons (or "HCFCs"), namely $CHCLF_2$, derived from chloroform and used to manufacture tetrafluoroethylene from which hexafluoropropylene in turn is derived, and $CH_3CCLF_2$, derived from methyl chloroform and used to manufacture vinylidene fluoride. Both of the HCFCs, as well as others, are believed to be less damaging to the earth's protective stratospheric ozone layer than chlorofluorocarbons ("CFCs'). However, because the HCFCs also transport chlorine to the stratosphere, a growing number of concerned environmentalists, scientists, and governments of various countries have suggested phasing out the use of HCFCs in the future or have proposed limiting their use to essential uses and then phasing out their use in the future (see "Chemical Regulation Reporter," December 1, 1989, P. 1187-1189, and "Chemical & Engineering News," December 4, 1989, p. 5,6).

In the patent literature, a number of processes have been disclosed for obtaining various fluoroolefins by pyrolyzing fluorocyclobutanes, said patents being discussed below.

U.S. Pat. No. 2,733,278 (Anderson et al.) discloses, inter alia, in Example VII, pyrolyzing 1,1,2,2-tetrafluorocyclobutane to produce a gaseous product comprising vinylidene fluoride, ethylene, and tetrafluoroethylene.

U.S. Pat. No. 3,652,691 (Bjornson et al.) discloses, inter alia, pyrolyzing octafluorocyclobutane under conditions such as to selectively produce a high yield of tetrafluoroethylene as the principal or desired product with substantially complete elimination of hexafluoropropylene production.

U.S. Pat. No. 3,662,009 (Hutchinson) discloses an "integrated unitary process" which comprises four steps which cooperate to produce the desired product, e.g., tetrafluoroethylene. The four steps illustrated in Example I are: (a) reacting tetrafluoroethylene with ethylene to form a partially fluorinated dimer product, 1,1,2,2-tetrafluorocyclobutane, by cyclodimerization, (b) fluorinating the dimer product to form an octafluorocyclobutane product, said fluorination being carried out electrochemically, preferably as disclosed in U.S. Pat. No. 3,511,760 (Fox et al.), i.e., a gas phase reaction between the partially fluorinated cyclic dimer with elemental fluorine; (c) pyrolyzing the octafluorocyclobutane to form the tetrafluoroethylene as the product of the process, and hexafluoropropylene; and (d) recycling to step (a) at least a portion of the tetrafluoroethylene product. In describing step (c), reference is made to the disclosure in said U. S. Pat. No. 3,652,691 (Bjornson et al.) which teaches pyrolysis conversion and temperature levels which should be observed in order to achieve high selectivity to the production of tetrafluoroethylene with substantially complete elimination of hexafluoropropylene production. In Example V of said U. S. Pat. No. 3,662,009, perfluorobutylene and tetrafluoroethylene are produced as pyrolysis products from butylene and tetrafluoroethylene.

U. S. Pat. No. 3,996,301 (Fozzard) discloses a process for producing vinylidene fluoride, comprising cracking tetrafluorocyclobutane to provide a cracked effluent containing vinylidene fluoride and unavoidably minor amounts of tetrafluoroethylene and ethylene, the cracked effluent, to which ethylene can be added, being subjected to dimerization and/or codimerization conditions under which tetrafluoroethylene is converted to tetrafluorocyclobutane, which can be recycled to the cracking zone, or to octafluorocyclobutane which can be separately cracked to yield tetrafluoroethylene, the dimerized effluent being separated to recover the desired vinylidene fluoride product.

Brazilian Patent No. 8,205,457, published August 23, 1983, discloses pyrolyzing tetrafluoroethylene, tetrafluoroethylene dimer, or a mixture of the two to produce hexafluoropropylene.

Briefly, in one aspect of this invention, a conductive solution of 1,1,2,2-tetrafluorocyclobutane in anhydrous liquid hydrogen fluoride is electrochemically fluorinated in an electrolytic cell by the "Simons process" in a continuous operation to produce octafluorocyclobutane, said fluorination being carried out at temperature and pressure conditions sufficient to convert the tetrafluorocyclobutane to a desired degree to octafluorocyclobutane and volatilize the same with minimal volatilization of incompletely fluorinated and unreacted cyclobutanes, the octafluorocyclobutane being removed from the cell as part of its gaseous effluent and thereby minimizing the concentration or residence of the octafluorocyclobutane in the electrolytic cell. The gaseous cell effluent can be cooled to condense and collect or recover the octafluorocyclobutane therefrom, which can then be pyrolyzed to produce hexafluoropropylene.

The "Simons process" or the "Simons electrochemical fluorination process" is a known, commercially practiced fluorination process carried out in an electrolytic cell (commonly referred to as a "Simons cell") to perfluorinate certain classes of organic compounds. An early patent describing such technology is U. S. Pat. No. 2,519,983 (Simons), which contains a drawing of a Simons cell and its appurtenances, and a description and photographs of laboratory and pilot plant cells appear at pages 416-418 of Volume 1 of "Fluorine Chemistry," edited by J. H. Simons, published in 1950 by Academic Press, Inc., New York. Further details of this fluorination technology will be described hereinbelow as applied in this invention.

Unlike the fluorination process disclosed in said U. S. Pat. No. 2,662,009 (Bjornson et al.) and 3,511,760 (Fox et al.) – now commonly known as the "Phillips process", e.g. see "Techniques of Chemistry," Vol. V, Chap. VII, Part III, by W. V. Childs, edited by N. L. Weinberg and B. V. Tilak (1982) -- which carries out the fluorination of an organic feed in the gas phase with elemental fluorine electrochemically generated from molten KF 2HF electrolyte, the Simons electrochemical fluorination is carried out in an electrolytic solution of the organic feed in essentially anhydrous liquid hydrogen fluoride, a fluorination in which elemental fluorine is not evolved. Thus, the Simons process is conceptually different from and not equivalent in operation to the Phillips process. We have surprisingly found that octafluorocyclobutane can be efficiently produced in good yields with high conversion of its precursor, while minimizing its ring-opening to form the acyclic perfluorobutane, $C_4F_{10}$, if the Simons cell is run at temperature and pressure conditions, e.g. 55° C at 35 to 45 psig, sufficient to boil or volatilize the octafluorocyclobutane but with minimal volatilization of tetrafluorocyclobutane and other incompletely fluorinated and unreacted cyclobutanes. The octafluoroc clobutane thus is thereby volatilized as it is formed and is removed as part of the gaseous overhead effluent, the concentration or residence time of the octafluorocyclobutane in the cell thus being minimized with consequently little of the ring-opened product, $C_4F_{10}$, being produced. For example, we have obtained by the above-described application of the Simons process octafluorocyclobutane yields of as much as 85-90 mole percent and greater than 99 mole percent total $C_4$ perfluorinated products.

In another aspect of this invention, the aforementioned electrochemical fluorination of tetrafluorocyclobutane by the Simons process is combined as a step together with several further steps: an upstream step of cycloaddition of tetrafluoroethylene with ethylene in the gas phase for the production of the tetrafluorocyclobutane feedstock used in the fluorination step; a downstream step of pyrolysis (or cracking) of the octafluorocyclobutane product to produce a pyrolysis product from which hexafluoropropylene and tetrafluoroethylene products are recovered; and a step in which some or all of said tetrafluoroethylene product is recycled to said cycloaddition step. This combination of steps can be considered as an integrated unitary continuous process to produce hexafluoropropylene as the desired product. Upon the process reaching steady state conditions, the tetrafluoroethylene requirements for the production of the tetrafluorocyclobutane will be satisfied to a significant degree or wholly by the tetrafluoroethylene recycled in the recycling step. Thus, the afore-described integrated process produces one of its own starting materials, viz., tetrafluoroethylene. The other starting materials are the ethylene used in the cycloaddition step and the hydrogen fluoride used in the fluorination step (said hydrogen fluoride eventually being to a significant degree or wholly the source of the fluorine content of the fluoroolefin products of the process).

In a further aspect of this invention, the aforementioned integrated process is modified by or further combined (without loss of its advantages) with a further step of pyrolysis (or cracking) of a portion of the cycloaddition product to produce a desired vinylidene fluoride product, such further combination of steps also being in a sense an integrated unitary process.

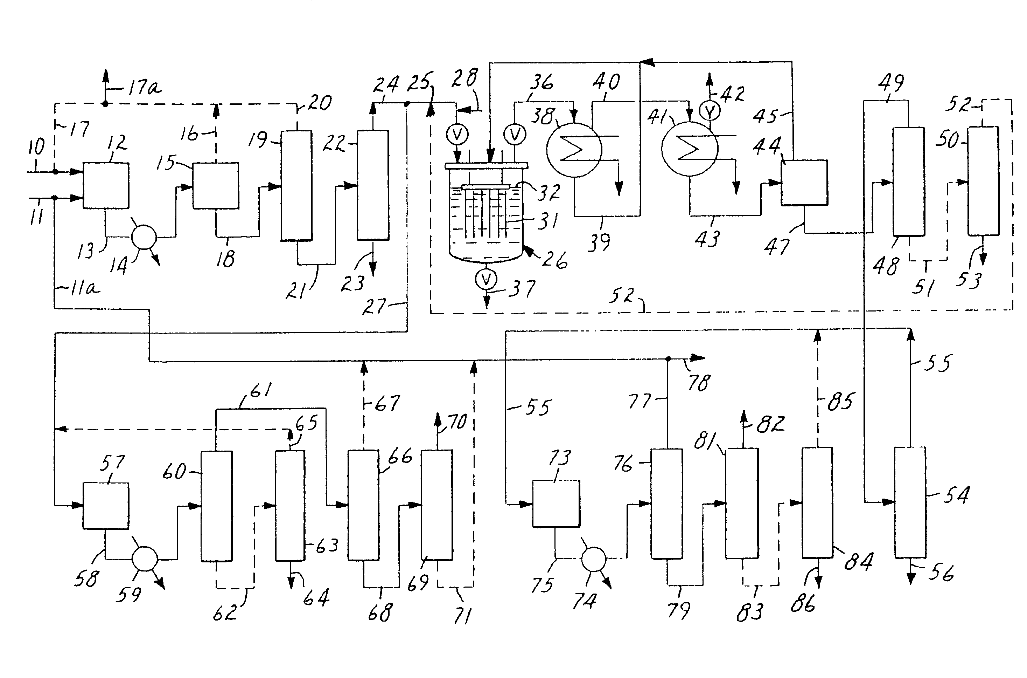

The accompanying drawing is a diagrammatic flow sheet (similar in some aspects to that of said U. S. Pat. No. 3,662,009) illustrating several embodiments of the invention.

Referring now to the drawing, in what can be regarded as the first step of the integrated unitary process of this invention, a stream 10 of ethylene (a relatively inexpensive commodity chemical) and a stream 11 of tetrafluoroethylene are initially introduced concurrently into cycloaddition reactor 12 where an exothermic, thermal cycloaddition (or dimerization) reaction is carried out in the gas phase to produce a partially-fluorinated fluorocyclobutane, viz. 1,1,2,2-tetrafluorocyclobutane, or c-$C_4F_4H_4$, as the principal product, as illustrated by the following equation:

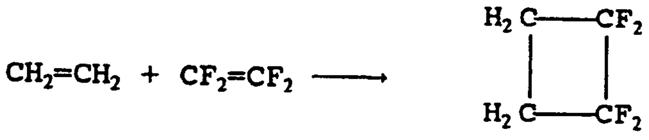

(1)

Generally, the relative mole ratio of ethylene to tetrafluoroethylene will be in the range of 0.3:1 to 50:1, preferably 5:1 to 20:1 and typically 10:1. A small amount of a fully fluorinated fluorocyclobutane, octafluorocyclobutane, or c-$C_4F_8$, is also formed by the homodimerization of tetrafluoroethylene:

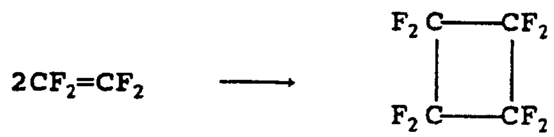

(2)

Oligomeric products of ethylene may also be formed in small amounts, particularly if the residence time is too long or the temperature is too high.

Reactor 12 can be of any suitable type, such as the tube and shell heat exchanger described in said U. S. Pat. No. 3,662,009, or other plug flow or turbulent flow tubular reactor, such as coils. A novel reactor for the cycloaddition reaction will be a continuously stirred tank reactor, in which the reaction temperature can be easier to control and a higher yield obtained of the desired dimer product with less by-product, such a type of reactor being described, for example, on pages 249-251, 269, and 270 of "An Introduction to Chemical Engineering Kinetics & Reactor Design," C. G. Hill, Jr., John Wiley & Sons, New York (1977).

The cycloaddition reaction can be carried out as described in said U. S. Pat. No. 3,662,009. Generally, the reactor will be operated at a temperature in the range of 150 to 600°C, preferably 300 to 450°C, and typically at about 400°C, a pressure in the range of 0.5 to 30 atmospheres, preferably 2 to 10 atmospheres, and typically at about 6 atmospheres, and a residence time in the range of 1 second to 10 minutes, preferably, at the preferred temperatures and pressures, of about 1 to 2 minutes. Short residence time leads to low conversion of feedstock and larger amounts of octafluorocyclobutane by-product, and long residence time produces larger amounts of oligomeric by-products.

The effluent from reactor 12 is passed via line 13 through cooler 14 and into separator 15 where a flash separation is effected to remove unreacted ethylene and small amounts of tetrafluoroethylene for recycle via lines 16, 17, and 10 to reactor 12. The essentially ethylene-free liquid product is withdrawn via line 18 from separator 15 and passed to into distillation column 19, any remaining ethylene and unreacted tetrafluoroethylene in the liquid product being withdrawn overhead via line 20 and recycled to reactor 12 via lines 17 and 10. Excess ethylene that is not needed to meet the stoichiometry of reactor 12 is bled off via line 17a along with low-boiling impurities that need to be purged. Liquid from column 19 is passed via line 21 to distillation column 22, from which high-boiling by-products can be withdrawn via line 23. The overhead withdrawn via line 24 from distillation column 22, comprising 1,1,2,2-tetrafluorocyclobutane and any octafluorocyclobutane by-product, is passed entirely or in part, via line 25 to a Simons electrolytic cell 26, the portion of the overhead not passed thereto being passed downstream via line 27 to a pyrolysis reactor (hereafter described). The cell feed comprising 1,1,2,2-tetrafluorocyclobutane in line 25 and anhydrous liquid hydrogen fluoride supplied via line 28 can be continuously fed separately or as a mixture (as shown) to cell 26 for electrolysis to produce the fully fluorinated cyclobutane, viz., octafluorocyclobutane. The electrochemical fluorination reaction carried out in Simons cell 26 is illustrated as follows:

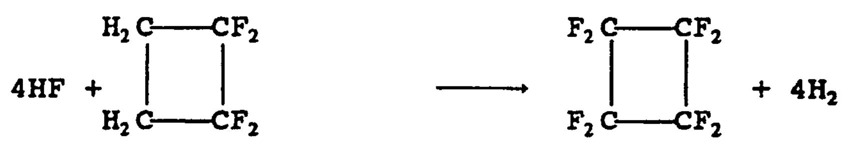

(3)

The Simons cell 26 comprises a cell body, typically made of carbon steel and usually provided with a cooling jacket, in which is suspended an electrode pack 31 comprising series of alternating and closely-spaced cathode plates (typically made of iron or nickel or nickel alloy) and anode plates (typically made of nickel), the plates being immersed in the current-conductive solution 32 of the partially fluorinated cyclobutane in essentially anhydrous liquid hydrogen fluoride. Gaseous cell effluent comprising the volatilized, fully fluorinated cyclobutane and volatilized hydrogen fluoride is withdrawn via valved-outlet line 36 and liquid drainings comprising hydrogen fluoride electrolyte are withdrawn via valved-outlet line 37 at the bottom of the cell 26. The electrolytic cell 26 is operated at conditions such as to volatilize the octafluorocyclobutane as it is produced so that it can be evolved or withdrawn from the cell via outlet line 36 and thereby minimize its concentration or residence in the cell where it otherwise would degrade to the acyclic fluorobutane by-product, $C_4F_{10}$. Generally, the temperature of the cell during the electrochemical fluorination is in the range of 20° to 70° C, preferably 50° to 60° C, and typically about 55°C, which is above the boiling point of octafluorocyclobutane (as well as hydrogen fluoride, which boils at about 20°C at ambient pressure). In operation, the cell is run at a pressure in the range of 0 to 65 psig and preferably 35 to 45 psig, but to ensure the volatilization of the octafluorocyclobutane, use of the higher pressures will, of course, require cell temperatures at the higher end of said temperature ranges.

The cell can be operated at average applied direct current cell voltages in the range of 4 to 9 volts, current density in the range of 10 to 100, preferably 20 to 80, mAmp/$cm^2$ of active anode surface (where the electrolysis takes place). The cell is operated either at constant current or constant voltage. The concentration of the 1,1,2,2-tetrafluorocyclobutane in the solution of anhydrous hydrogen fluoride generally will be 5 to 20 weight percent. After initiation of electrolysis, the conductivity of the solution of organic substrate in anhydrous liquid hydrogen is normally adequate and the electrolysis is conducted in the absence of added conductivity additive, but conventional electrolyte conductivity additive may be added to increase conductivity if desired, such additives being used in the amount, for example, of 1 to 20 percent of the weight of the 1,1,2,2-tetrafluorocyclobutane. The additive can be, for example, sodium fluoride, acetic anhydride, or an organic sulfur-containing compound such as described in U. S. Pat. Nos. 3,028,321 (Danielson), 3,692,643 (Holland), and 4,739,103 (Hansen).

Other details of the Simons electrochemical fluorination and cell will be omitted here in the interest of brevity, and the disclosures of such technology in the above cited references to such technology can be referred to for such detail, which references are incorporated herein for such purpose.

The reactor gaseous effluent comprising octafluorocyclobutane, hydrogen, hydrogen fluoride, and other gaseous products (minimal amounts of tetrafluorocyclobutane and other incompletely fluorinated cyclobutanes) is withdrawn from the top of the reactor 26 and passed via line 36 through a condenser 38. The resulting condensate, comprising hydrogen fluoride, tetrafluorocyclobutane and other incompletely fluorinated cyclobutanes, is recycled to reactor 26 via line 39. The uncondensed gaseous or volatile portion is passed via line 40 to condenser 41 for removal via line 42 of hydrogen, uncondensed octafluorocyclobutane and decafluorobutane from the condensible portion of stream 41. The condensible portion of stream 41 containing octafluorocyclobutane product, decafluorobutane, some partially fluorinated materials, and hydrogen fluoride is fed via line 43 to decanter 44 where said decafluorobutane, octafluorocyclobutane, and partially fluorinated materials are separated from the hydrogen fluoride phase, which can be returned via line 45 to reactor 26. The octafluorocyclobutane product, stream 47, passes to distillation column 48, where octafluorocyclobutane and by-product decafluorobutane are separated overhead as stream 49. Unreacted tetrafluorocyclobutane, other partially fluorinated materials, and high boilers are optionally fed to distillation column 50 as stream 51. Tetrafluorocyclobutane is taken off the top of column 50 and optionally recycled to reactor 26 as stream 52; high boiling bottoms are removed as stream 53. Alternatively, stream 51 can be recycled to column 22. Stream 49 from column 48 is fed to distillation column 54 where the octafluorocyclobutane is taken off the top as stream 55, and decafluorobutane is removed as bottoms stream 56. Using no selective recycle of the condensate to the Simons cell and continuous cell operation (continuous feed of hydrogen fluoride and tetrafluorocyclobutane with product removal effected by use of the decanter through which all condensate is passed) resulted in a steady state product takeoff having the following approximate composition: 24 mole percent tetrafluorocyclobutane, 6-7 mole percent of fluorinated material with intermediate degree of fluorination, 59-63 mole percent octafluorocyclobutane, 7.0-9.5 mole percent decafluorobutane, and less than 1 mole percent perfluoropropane. Recycling condensate from the first condenser 38 and controlling the temperature of the coolant to that condenser at 0° lowered the level of unconverted tetrafluorocyclobutane in the product stream to less than 8 mole percent (5 weight percent) without altering the distribution of perfluorinated products.

Lowering the level of unconverted tetrafluorocyclobutane in the octafluorocyclobutane product stream is the important objective of recycling that portion of the process stream condensed at or above a selected temperature. This temperature is most easily optimized empirically. This optimum will be the temperature at which the rate of passage of uncondensed octafluorocyclobutane product in the hydrogen- and hydrogen fluoride-containing process stream from the cell is equal to or just slightly greater than its rate of production in the cell. Because of the high solubility of perfluoro products and the tetrafluorocyclobutane in cold hydrogen fluoride, this temperature will be somewhat higher than that calculated from the equilibrium vapor pressure curves for the product in the absence of the hydrogen fluoride.

The optimum temperature for the first condenser 38 is that temperature just high enough to let perfluorinated cell products pass to the product collection system at the rate they are formed, which prevents build-up of perfluorinated material in the cell 26.

The pyrolysis of a portion of the 1,1,2,2-tetrafluorocyclobutane, whose production is described above, to produce vinylidene fluoride takes place in a pyrolysis reactor 57 such as that described in U. S. Pat. Nos. 2,462,345, 2,733,278, and 3,996,301. The pyrolysis is illustrated as follows:

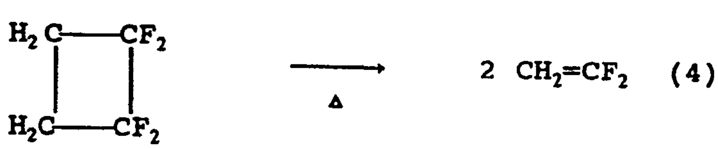

2 $CH_2=CF_2$ (4)

Preferably, pyrolysis reactor 57, into which tetrafluorocyclobutane stream 27 is fed, comprises a small diameter tube which can be heated in any suitable manner, e.g., radiation from electrical coils, burning of fuel, etc. The reactor can be operated at a temperature in the range of 700 to 900°C, preferably about 750°C, a pressure of about 0.5 to 4 atmospheres, preferably 1 atmosphere, and a residence time of 0.05 to 5 seconds, preferably about 1 second.

The gaseous product stream 58 is fed through cooler 59 and then fed to distillation column 60, where the lower boiling compounds are sepal-ated from the unconverted feed as overhead stream 61. Unconverted bottoms stream 62, comprising tetrafluorocyclobutane and other high boiling impurities, optionally is further purified in distillation column 63 to remove trace high boiling impurities as bottoms stream 64 before optionally being recycled via stream 65 to pyrolysis reactor 57. The predominantly ethylenic stream 61, comprising vinylidene fluoride, tetrafluoroethylene, and ethylene, is fed to distillation column 66, where ethylene is removed as stream 67, which optionally is recycled to cycloaddition reactor 12 via line 11a. The bottoms product, stream 68, is fed to distillation column 69, where the desired vinylidene fluoride is obtained as overhead product stream 70, and the bottoms residue; primarily tetrafluoroethylene, optionally is recycled to cycloaddition reactor 12 via stream 71 and 11a.

Stream 55, containing purified octafluorocyclobutane, is fed to pyrolysis reactor 73, wherein it is pyrolyzed to form products comprising predominantly tetrafluoroethylene and hexafluoropropylene. The pyrolysis reaction is illustrated as follows:

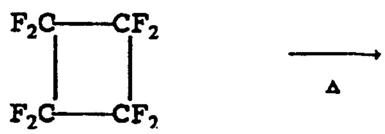

$CF_2=CF_2 + CF_2=CFCF_3$ (5)

The preferred form for reactor 73 is the same as reactor 57. In one embodiment of the invention, pyrolysis reactor 73 is operated at about 85°C, a pressure of about 1 atmosphere, and a residence time of about 0.2 seconds. The pyrolysis effluent is fed into cooler 74 as stream 75 and then to distillation column 76, where tetrafluoroethylene is removed from the top of the column as stream 77. Although optional if a separate source of tetrafluoroethylene were provided, tetrafluoroethylene stream 77 preferably is sent to reactor 12 via line 11a and generally constitutes the primary source of tetrafluoroethylene employed in the unified process of this invention. Alternatively, a portion of the tetrafluoroethylene as stream 78 can be removed as a product of the process. The bottoms from distillation column 76 are removed as stream 79 and fed into distillation column 81. The desired hexafluoropropylene product of the process is taken from the top of column 81 as stream 82, and the bottoms stream 83 optionally is fed to distillation column 84, where unreacted octafluorocyclobutane may be removed from the top of the column as stream 85 and optionally recycled to pyrolysis reactor 73 via line 55. The bottoms stream 86 from column 84 consists mainly of perfluorinated butenes, which can also be recycled to pyrolysis reactor 73.

Material balances illustrating the foregoing are set forth in Tables I and II, the streams whose compositions depicted in the drawing.

Table I is a material balance for the above-described process where the product of the process is only hexafluoropropylene, $C_3F_6$, (removed by product line 82) and desired production thereof is 100 Kg/hour. In that process, all of the tetrafluorocyclobutane, c-$C_4F_4H_4$ removed as overhead from distillation column 22 is passed via lines 24, 25 to the Simons electrochemical fluorination cell 26 (that is, none of said overhead is passed via lines 24, 27 to pyrolysis reactor 57 where vinylidene fluoride is otherwise co-currently produced, as described below in connection with Table II). The data of Table I assumes that the yield of tetrafluorocyclobutane from the cycloaddition of ethylene and tetrafluoroethylene in reactor 12 is 94%, based on recycled tetrafluoroethylene supplied via line 11a. The yield of octafluorocyclobutane from the Simons cell 26 is assumed to be 87%, and the recovery of all fluorinated products is assumed to be 97%. The pyrolysis of the octafluorocyclobutane feed in pyrolysis reactor 73 is carried out under conditions, including residence time of the feed in the reactor, such that 60% of the feed is converted to a conversion product (stream 75) made up of 65% tetrafluoroethylene (all of which is recycled via lines 27, 11a to cycloaddition reactor 12), 30% hexafluoropropylene, and 5% higher boiling materials and butenes. In so operating the process for production of hexafluoropropylene, 3.4 moles of tetrafluoroethylene are recycled for each mole of hexafluoropropylene produced.

Table II is a material balance for the above-described process where the products of the process are hexafluoropropylene and vinylidene fluoride (which is removed by product line 70). The material balance of Table II is for 100 Kg/hour of hexafluoropropylene product and 150 Kg/hour of vinylidene fluoride product. For this two-product process, the tetrafluorocyclobutane recovered as overhead via line 24 from distillation column 22 is split into stream 25 (supplied as feed to Simons cell 26) and stream 27 (supplied as pyrolysis reactor 57). The

conditions set for pyrolysis reactor 73 include a shorter residence time (than that set for the process illustrated in Table I) and other conditions such as to result in a 40% conversion of octafluorocyclobutane to a stream (line 75) made up of 83.5% tetrafluoroethylene (all of which is recycled to cycloaddition reactor 12 via line 11a), 15% hexafluoropropylene, and 1.5% of high boilers and butenes. The pyrolysis of tetrafluorocyclobutane in pyrolysis reactor 57 is carried out under optimum conditions for production of vinylidene fluoride, which for purposes of the material balance of Table II results in a 60% conversion of the feed in reactor 57, 95% of the reaction product withdrawn therefrom via line 58 being made up of $C_2$-olefins, of which 90% is vinylidene fluoride product and 10% is a mixture of ethylene and tetrafluoroethylene (which is recycled via lines 67, 11a to cycloaddition reactor 12), the other 5% of stream 58 being higher boiling by-products and butenes. In so operating the process illustrated by the material balance of Table II, 8.3 moles of tetrafluoroethylene are recycled for one mole of hexafluoropropylene product and 3.5 moles of vinylidene fluoride product.

As an example of the electrochemical process of this invention carried out in a Simons cell, a 50-ampere cell of the type described in U. S. Pat. No. 2,713,593 was charged with 1500 g liquid anhydrous hydrogen fluoride and 30 g dimethyl disulfide. The cell was started and the 1,1,2,2-tetrafluorocyclobutane was fed continuously to the cell at the rate of 14.0 g per hour. The cell was operated at a reactor pressure of 45 psig and a temperature of 61°C. At steady state, the voltage was typically about 6.45 volts and the current typically about 21 amps.

Two condensers, like condensers 38 and 41, the first at 0°C and the second at -45°C, equipped with a product decanter, like decanter 44, were used in series to partially condense hydrogen fluoride and products from the fluorination. All condensate, comprising principally hydrogen fluoride and tetrafluorocyclobutane, from the first condenser was returned directly to the cell in order to minimize the amount of tetrafluorocyclobutane removed with the octafluorocyclobutane product stream. During the run, a total of 19.7 Kg of tetrafluorocyclobutane was fed to the cell and 23.9 Kg of crude cell product (approximately 80% of the fluorinated product from the cell) was collected in the decanter from the -45°C condenser. The remainder escaped through the vent 42. The vented product and the condensed product were analyzed by gas chromatography. Typical crude product (including the collected liquid and the material vented from the system) under the aforesaid operating conditions comprised by weight approximately 5% unreacted tetrafluorocyclobutane, 5 to 7% hydrides (incompletely fluorinated intermediates), 74 to 76% octafluorocyclobutane, and 13 to 14% decafluorobutane.

TABLE 1

MATERIAL BALANCE OF COMPONENTS (in Kg/hr) FOR PRODUCTION OF HEXFLUOROPROPYLENE AS PRODUCT OF PROCESS

| | STREAM | | | | | | | | | | | | | |
|---|---|---|---|---|---|---|---|---|---|---|---|---|---|---|
| Component | 10 | 17a | 23 | 25 | 28 | 42 | 53 | 55 | 56 | 55a | 82 | 85 | 86 | 24 |
| $C_2H_4$ | 58 | 3 | | | | | | | | | | | | |
| $C_2F_6$ | | 0.2 | | 0.2 | | | | | | | | | | |
| $C_2F_4$ | | | | 213 | | | | | | | | | | |
| $C_3F_6$ | | | | | | | | | | | 100 | | | |
| $c-C_4F_4H_4$ | | | | | | | | | | | | | | 230 |
| $c-C_4F_8$ | | | | | | 14 | | 328 | | 546 | | 219 | | |
| $C_4F_{10}$ | | | | | | 3 | | | 47 | | | | | |
| Butenes | | | | | | | | | | | | | 15 | |
| Heavies | | | 15 | | | | 0.5 | | | | | | | |
| HF | | | | | 157 | 3 | | | | | | | | |
| $H_2$ | | | | | | 14 | | | | | | | | |

TABLE II

**MATERIAL BALANCE OF COMPONENTS (in Kg/hr) FOR PRODUCTION OF HEXAFLUOROPROPYLENE AND VINYLIDENE FLUORIDE AS PRODUCTS OF PROCESS**

STREAM

| Component | 10 | 17a | 23 | 25 | 55 | 28 | 42 | 53 | 55b | 56 | 55a | 77 | 82 | 85 | 86 | 65 | 67 | 70 | 71 | 64 |
|---|---|---|---|---|---|---|---|---|---|---|---|---|---|---|---|---|---|---|---|---|
| $C_2H_2$ | 159 | 3 | | | | | | | | | | | | | | | 3 | | | |
| $C_2F_6$ | | 0.2 | | | | | | | | | | | | | | | | | | |
| $C_2F_4$ | | | | | | | | | | | | 556 | | | | | | | 12 | |
| $C_2F_2H_2$ | | | | | | | | | | | | | | | | | | 150 | | |
| $C_3F_6$ | | | | | | | | | | | | | 100 | | | | | | | |
| $c-C_4F_4H_4$ | | | | 466 | 174 | | | | | | | | | | | | | | | 46 |
| $c-C_4F_8$ | | | | | | | 29 | | 666 | | 1665 | | | 999 | | | | | | |
| $C_4F_{10}$ | | | | | | | 5 | | | 96 | | | | | | | | | | |
| Butenes | | | | | | | | | | | | | | | 10 | | | | | |
| Heavies | | | 40 | | | | | 1 | | | | | | | | 9 | | | | |
| HF | | | | | | 320 | 6 | | | | | | | | | | | | | |
| $H_2$ | | | | | | | 29 | | | | | | | | | | | | | |

Various modifications and variations of this invention will become apparent to those skilled in the art without departing from the scope and spirit of this invention.

## Claims

1. A process for the production of octafluorocyclobutane, which comprises fluorinating by the Simons electrochemical fluorination process in an electrochemical cell a solution of 1,1,2,2-tetrafluorocyclobutane in anhydrous liquid hydrogen fluoride at temperature and pressure conditions sufficient to convert to a desired degree the tetrafluorocyclobutane to octafluorocyclobutane and volatilize the same with minimal volatilization of incompletely fluorinated and unreacted cyclobutanes.

2. A process for the production of hexafluoropropylene, which comprises (a) reacting ethylene and tetrafluoroethylene in a cycloaddition reactor to form 1,1,2,2-tetrafluorocyclobutane, (b) fluorinating by the Simons electrochemical fluorination process in an electrochemical fluorination cell a solution of at least a portion of said 1,1,2,2-tetrafluorocyclobutane in anhydrous liquid hydrogen fluoride at temperature and pressure conditions sufficient to convert the tetracyclobutane to a desired degree to octafluorocyclobutane and volatilize the same with minimal volatilization of incompletely fluorinated and unreacted cyclobutanes, (c) removing from said cell its gaseous effluent containing volatilized octafluorocyclobutane, tetrafluorocyclobutane, and hydrogen fluoride, (d) cooling said gaseous effluent so as to separately condense and collect a first liquid comprising principally octafluorocyclobutane and a second liquid comprising principally hydrogen fluoride and tetrafluorocyclobutane, (e) recycling said second liquid to said cell, (f) pyrolyzing said first liquid to form hexafluoropropylene and tetrafluoroethylene, (g) recovering said hexafluoropropylene as the product of the process, and (h) recycling to said cycloaddition reactor at least a portion of said tetrafluoroethylene formed in said pyrolyzing step.

3. A process for the production of hexafluoropropylene and vinylidene fluoride, which comprises (a) reacting ethylene and tetrafluoroethylene in a cycloaddition reactor to form 1,1,2,2-tetrafluorocyclobutane, (b) fluorinating by the Simons electrochemical fluorination process in an electrochemical fluorination cell a solution of at least a portion of said 1,1,2,2-tetrafluorocyclobutane in anhydrous liquid hydrogen fluoride at temperature and pressure conditions sufficient to convert the tetrafluorocyclobutane to a desired degree to octafluorocyclobutane and volatilize the same with minimal volatilization of incompletely fluorinated and unreacted cyclobutanes, (c) removing from said cell its gaseous effluent containing volatilized octafluorocyclobutane, tetrafluorocyclobutane, and hydrogen fluoride, (d) cooling said gaseous effluent so as to separately condense and collect a first liquid comprising principally octafluorocyclobutane and a second liquid comprising principally hydrogen fluoride and tetrafluorocyclobutane, recycling said second liquid to said cell, (e) pyrolyzing the said first liquid to form hexafluoropropylene and tetrafluoroethylene, (f) recovering said hexafluoropropylene as a product of the process, (g) recycling to said cycloaddition reactor at least a portion of said tetrafluoroethylene formed in said pyrolyzing step, (h) pyrolyzing another portion of said tetrafluorocyclobutane to pyrolysis to form vinylidene fluoride, and (i) recovering the latter as another product of the process.

4. In a process for the production of 1,1,2,2-tetrafluorocyclobutane by reacting ethylene and tetrafluoroethylene in a cycloaddition reactor, the improvement comprising using as said reactor a continuously stirred tank reactor.

5. The process of Claim 2, wherein said cycloaddition reactor is a continuously stirred tank reactor.